# EUROPEAN PATENT APPLICATION

(11) **EP 2 389 932 A1**
(43) Date of publication of application: **30.11.2011**
(21) Application number: 10005571.4
(22) Date of filing: 28.05.2010
(51) Int. Cl.: A61K 31/192, A61P 15/02, A61P 9/14, A61P 25/24, A61P 43/00

(54) **Compositions for use in genetic disorders comprising 4-phenyl-butyric acid and its salts**

(71) Applicant: Lunamed AG, 7000 Chur (CH)
(72) Inventor: Truog, Peter, 7000 Chur (CH)
(74) Representative: Peters, Hajo

(57) **Abstract**

The present invention refers to pharmaceutical compositions comprising the histone hyperacetylating agent phenyl-butyric acid, or a physiologically acceptable salt thereof for use in the treatment of a genetic disorder, especially of an epigenetic disorder, like treatment of depression, vaginitis and varicosis or the prevention of Sudden Infant Death Syndrome with a genetic disorder background.

## Description

### Field of the invention

The present invention refers to pharmaceutical compositions comprising phenyl-butyric acid (a histone hyperacetylating agent), a derivative or a physiologically acceptable salt thereof for use in the treatment of a genetic disorder, especially of an epigenetic disorder, like treatment of depression, vaginitis and varicosis or the prevention of Sudden Infant Death Syndrome with a genetic disorder background.

### Background of the invention

Genetic Disorders are commonly referred to as disorders that are caused by and based on changes in one or more genes but in the context of this invention especially refers to changes to their accessibility, phenotype or expression. Thus the focus of this invention is lying on epigenetic disorders such as thalessemia, or urea cycle disorders or which - at least in part - are the cause for many highly diverse diseases and symptoms such as varicosis, depression or vaginitis. Many of these diseases have fatal consequences on those suffering from them, especially in young children whose disposition remains undiscovered until the - sometimes grave ― consequences of the hereditary and/or developing disorder come to light. It seems plausible that many cases of the dreaded Sudden Infant Death Syndrome might rest in undiscovered genetic disorders. There is currently no cure and treatment properly offered for such disorders and very few attempts in treating those suffering from these disorders are reported.

Accordingly, it was the objective of the present invention to provide a new form of treatment for genetic disorders or of symptoms or diseases wherein this genetic disorder manifests itself including such up to now only in rare cases treatable diseases/symptoms like vaginitis, depression or varicosis and thus also for prevention of - at least in part of - the Sudden Infant Death Syndrome.

It has now surprisingly been found that administration of the histone hyperacetylating agent phenyl-butyric and related compounds results in therapeutical effects on genetic disorders or the symtoms and diseases they cause, including strongly ameliorating symptoms up to effects that could be described as gene repair.

Accordingly, the invention refers to a pharmaceutical composition comprising a histone hyperacetylating agent for use in the treatment of a genetic disorder. Preferably the genetic disorder is an epigenetic disorder. Preferably the histone hyperacetylating agent is a histone deacetylase inhibitor, preferably is selected from the group consisting of Trichostatin A and Trichostatin C; phenyl-butyrate; the group consisting of Oxamflati, Trapoxin A, FR901228, Apicidin, HC-Toxin, WF27082, and Chlamydocin; the group consisting of Salicylihydroxamic Acid, Suberoylanilide Hydroxamic Acid, and Azelaic Bishydroxamic Acid; the group consisting of Azelaic-I-Hydroxamate-9-Anilide, M-Carboxycinnamic Acid Bishydroxamide, 6-(3-Chlorophenylureido)carpoicHydroxamic Acid, MW2796, and MW2996; the group consisting of Sodium Butyrate, Isovalerate, Valerate, 4-Phenylbutyrate, Phenylbutyrate, Propionate, Butrymide, Isobutyramide, Phenylacetate, 3-Bromopropionate, and Tributyrin; the group consisting of MS-27-275 and the 3'-amino derivative thereof; or the group consisting of Depudecin and Scriptaid or is Vorinostat or other related Hydroxamic Acids; or from those new histone deacetylase inhibitors listed in the article "New patented histone deacetylase inhibitors" by Wang H. and Dymock BW (Expert Opin. Ther. Patents (2009) 19(12):1727-1757) like AR-42, belinostat (PXD101), benzamides, chidamide (CS055/HBI8000), CHR-2845, CUDC-101, cyclic peptides, depsipeptide, entinostat (MS-275), givinostat (ITF2357), hydroxamates, JNJ26481585, KD-5170, mocetinostat (MGCD0103), panobinostat (LBH589), PCI-24781 (CRA-024781), resminostat (4SC-201, BYK408740), romidepsin (FK228), SB939, or vorinostat (SAHA).

Preferably though, the invention refers to a pharmaceutical composition comprising an active ingredient A or to active ingredient A itself selected from a phenyl-butyric acid, a derivative or a physiologically acceptable salt thereof for use in the treatment of a genetic disorder. Most preferably the genetic disorder is an epigenetic disorder.

Phenyl - butyric acid, especially 4-Phenyl- butyric Acid is a histone hyperacetylating agent that is known as a pharmaceutically active ingredient for many years.

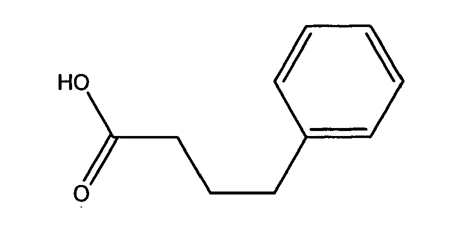

It may form salts and the most common salts known are salts with alkali and earth-alkali metals, like lithium, sodium, magnesium or calcium, with sodium being the most common.

*"Genetic disorder"* is defined as disorders that are caused by and/or based on changes in one or more genes that are inherited from at least one of the parents. Examples include urea cycle disorders, thalassemia, but also the embodiments of diseases or symptoms such as varicosis, vaginitis, depression or Sudden Infant Death Syndrome etc., that are based on or caused by these changes. A list of relevant disorders or related diseases and symptoms follows below.

*"Epigenetic" or "epigenetic disorder"* is defined as an inherited change in phenotype or gene expression which is not caused by changes to the gene sequence but is caused other mechanism/non-genetic factors.

*"Treatment of a Genetic disorder"* or *"Treatment of an epigenetic disorder"* is defined as a therapeutical effects on the genetic disorders, including ameliorating, strongly ameliorating or even curing of the related/caused symptoms, curing the related/caused disease by removing the cause up to gene repair removing the genetic or epigenetic disorder. Thus it would also apply to a treatment by curing the genetic disorder (also if this disorder is only the underlying cause for a disease or a symptom). It might also apply to prophylactic approaches and thus *"Treatment of* a *Genetic disorder"* or *"Treatment of an epigenetic disorder"* would also encompass the prevention of a disease or symptom caused by a genetic disorder or epigenetic disorder.

Preferably the pharmaceutical composition is used according to the invention for a genetic disorder being selected from or that manifests itself in symptoms or diseases selected from urea cycle disorders, thalassemia, cystic fibrosis, rheumatoid arthritis, Siogren' s syndrome, uveitis, varicosis, polymyositis, and dermatomyositis, arteriosclerosis, amyotrophic lateral sclerosis, asociality, affective disorders, systemic lupus erythematosus. It rest also in cardiovascular and neurological diseases, type II diabetes, neurodegenerative diseases, Rubinstein-Taybi-Syndrome, Rett syndrome, Friedreich's ataxia, Huntingdon's disease, multiple sclerosis, depression. It might also include mucositis, skin/mucosal itching, degenerative diseases of the eye, eating disorders and obesity, drug induced weight gain, pruritus, alcoholism, grey hair, hair loss, cardiac injury, lack of neuronal growth, osteoporosis, bone and joint diseases, epithelial damage, desmosis, Parkinson's disease, myelodysplastic syndrome, fibrotic lung diseases, hepatic encephalopathies, infections by human papilloma virus (HPV) or autoimmune diseases or also vaginitis or Sudden Infant Death Syndrome. In general, as already stated in the definition of "genetic disorder" the use refers only to diseases/symptoms listed in this paragraph as far as their cause rests in a genetic disorder, especially in an epigenetic disorder.

Genetic disorders in general include the 22q11.2 deletion syndrome, Angelman Syndrome, Canavan disease, celiac disease, Charcot-Marie-Tooth disease, Color blindness, Cri du chat, Down syndrome, Cystic fibrosis, Duchenne muscular dystrophy, Haemophilia, Klinefelter's syndrome, neurofibromatosis, phenylketonuria, Prader-Willi syndrome, sickle cell disease, Tay-Sachs disease, Turner syndrome etc.

Accordingly, in one embodiment the pharmaceutical composition is used according to the invention for a genetic disorder being selected from or manifests itself in symptoms or diseases selected from urea cycle disorders, thalassemia, cystic fibrosis, rheumatoid arthritis, Siogren' s syndrome, uveitis, polymyositis, and dermatomyositis, arteriosclerosis, amyotrophic lateral sclerosis, asociality, affective disorders, systemic lupus erythematosus, immune response, varicosis, vaginitis, including chronic recurrent yeast vaginitis, depression or Sudden Infant Death Syndrome, preferably selected from depression, varicosis, or Sudden Infant Death Syndrome.

One preferred embodiment "P" relates to a pharmaceutical composition according to the invention, comprising an active ingredient A or to the active ingredient A itself selected from a phenyl-butyric acid or a physiologically acceptable salt thereof for use in the treatment of a genetic disorder by administration of between 80 to 4000 mg/day, or 200 to 2000 mg/day or of a maximum of 1000 mg/day of the active ingredient A, whereas the genetic disorder is an epigenetic disorder.

Preferably, in the pharmaceutical composition "P" used according to the invention the treatment is applied by oral administration of the pharmaceutical composition, preferably is applied by once or twice daily oral administration of the pharmaceutical composition. It is also preferable if the treatment is applied to a child, preferably is applied to a child (weighing less than 20 kg) by administration of an amount of active ingredient A less than 450 mg/kg/day.

Alternatively, in the pharmaceutical composition "P" used according to the invention the treatment is applied by administration of a maximum of between 750 and 250 mg/day of active ingredient A, preferably of between 600 and 400 mg/day of active ingredient A, most preferably of approximately or a maximum of 500 mg/day of active ingredient A.

Preferably, in the pharmaceutical composition "P" used according to the invention the active ingredient A is 4-phenyl-butyric acid or a physiologically acceptable salt thereof, preferably is a physiologically acceptable salt of 4-phenyl-butyric acid, more preferably is a physiologically acceptable salt of 4-phenyl-butyric acid of the earth or earth alkaline type, most preferably is a lithium, sodium, magnesium or calcium, preferably the sodium salt of 4-phenyl-butyric acid.

Most preferably in the pharmaceutical composition "P" used according to the invention the pharmaceutical composition is a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC). Other options could include pharmaceutical composition (including "P") being oral pharmaceutical unit dosage forms with an enteric coating or being based on nanoparticles to ensure a constant blood level of the active ingredient A. Very preferred embodiments of the oral pharmaceutical composition "P" to be used according to the invention and comprising 4-phenyl-butyric acid or a physiologically acceptable salt thereof are disclosed in EP1427396 (WO2003/22253), the content of which forming herewith part of the description/disclosure of the current invention. In using this oral pharmaceutical composition, the active ingredient A is applied twice daily with 40 to 2000 mg/day, or 100 to 1000 mg/day or twice daily with 250 mg/day of the active ingredient A (like 4-phenyl-butyric acid or sodium phenylbutyrate).

In a most preferred embodiment, the invention relates to a pharmaceutical composition according to the invention comprising an active ingredient A selected from a phenyl-butyric acid or a physiologically acceptable salt thereof for use in the treatment of a genetic disorder by administration of between 80 to 4000 mg/day, or 200 to 2000 mg/day or of a maximum of 1000 mg/day of the active ingredient A, wherein the genetic disorder is an epigenetic disorder.

One of the very much preferred aspects/embodiments of this invention relates to a histone hyperacetylating agent or a pharmaceutical composition comprising a histone hyperacetylating agent for use in the treatment of depression. The use is focused/limited to the treatment of Depression caused by the consequences of a genetic disorder, especially an epigenetic disorder.

*"Depression"* is an impairment of affectivity. It is a state of low mood and aversion to activity.

*"Treatment of Depression"* is defined as a therapeutical effect on the depression, including ameliorating, strongly ameliorating or even curing the symptoms, including e.g. the improvement or stabilization of the mood. Thus, it could also include curing the disease by removing the cause up to gene repair like removing the epigenetic disorder. It might also apply to prophylactic approaches and thus *"Treatment of* a *Depression"* would also encompass the prevention of depression caused by a genetic disorder or epigenetic disorder.

In one embodiment the histone hyperacetylating agent itself or being comprised in the pharmaceutical composition used in the treatment of depression is a histone deacetylase inhibitor, preferably is selected from the group consisting of Trichostatin A and Trichostatin C; phenyl-butyrate; the group consisting of Oxamflati, Trapoxin A, FR901228, Apicidin, HC-Toxin, WF27082, and Chlamydocin; the group consisting of Salicylihydroxamic Acid, Suberoylanilide Hydroxamic Acid, and Azelaic Bishydroxamic Acid; the group consisting of Azelaic-I-Hydroxamate-9-Anilide, M-Carboxycinnamic Acid Bishydroxamide, 6-(3-Chlorophenylureido)carpoicHydroxamic Acid, MW2796, and MW2996; the group consisting of Sodium Butyrate, Isovalerate, Valerate, 4-Phenylbutyrate, Phenylbutyrate, Propionate, Butrymide, lsobutyramide, Phenylacetate, 3-Bromopropionate, and Tributyrin; the group consisting of MS-27-275 and the 3'-amino derivative thereof; or the group consisting of Depudecin and Scriptaid or is Vorinostat or other related Hydroxamic Acids; or from those new histone deacetylase inhibitors listed in the article "New patented histone deacetylase inhibitors" by Wang H. and Dymock BW (Expert Opin. Ther. Patents (2009) 19(12):1727-1757) like AR-42, belinostat (PXD101), benzamides, chidamide (CS055/HBI8000), CHR-2845, CUDC-101, cyclic peptides, depsipeptide, entinostat (MS-275), givinostat (ITF2357), hydroxamates, JNJ26481585, KD-5170, mocetinostat (MGCD0103), panobinostat (LBH589), PCI-24781 (CRA-024781), resminostat (4SC-201, BYK408740), romidepsin (FK228), SB939, or vorinostat (SAHA).

In a very preferred embodiment the histone hyperacetylating agent itself or being comprised in the pharmaceutical composition used in the treatment of depression is an active ingredient A selected from a phenyl-butyric acid, derivative or physiologically acceptable salt thereof, preferably being selected from 4-phenyl-butyric acid or a physiologically acceptable salt thereof, more preferably being a physiologically acceptable salt of 4-phenyl-butyric acid, more preferably being a physiologically acceptable salt of 4-phenyl-butyric acid of the earth or earth alkaline type, most preferably is a lithium, sodium, magnesium or calcium, preferably the sodium salt of 4-phenyl-butyric acid.

In another preferred embodiment of the histone hyperacetylating agent or the pharmaceutical composition comprising it used in the treatment of depression the treatment is done by treating the patient by administration of an amount of active ingredient A ingredient A of A of less than 9.9 g/m²/day, or by .

In another preferred embodiment of the histone hyperacetylating agent or the pharmaceutical composition comprising it, used in the treatment of depression the treatment is done by treating the patient by oral administration of active ingredient A or the pharmaceutical composition, preferably by once or twice daily oral administration of active ingredient A or the pharmaceutical composition.

A highly important aspect of the invention rests in that the use of the active ingredient A (the histone hyperacetylating agent) or of the pharmaceutical composition comprising it, is done in a way to ensure a constant level (concentration) of the active ingredient A (phenyl-butyric acid, derivative or physiologically acceptable salt thereof) in the blood.

Accordingly, in another preferred embodiment the active ingredient A (the histone hyperacetylating agent) used in the treatment of depression is comprised in a pharmaceutical composition being a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC). Other options could include pharmaceutical composition being oral pharmaceutical unit dosage forms with an enteric coating or being based on nanoparticles to ensure a constant blood level of the active ingredient A. Very preferred embodiments of the oral pharmaceutical composition to be used according to the invention and comprising 4-phenyl-butyric acid or a physiologically acceptable salt thereof are disclosed in EP1427396 (WO2003/22253), the content of which forming herewith part of the description/disclosure of the current invention. In using this oral pharmaceutical composition, the active ingredient A is applied twice daily with 40 to 2000 mg/day, or 100 to 1000 mg/day or twice daily with 250 mg/day of the active ingredient A (like 4-phenyl-butyric acid or sodium phenylbutyrate).

Another of the very much preferred aspects/embodiments of this invention relates to a histone hyperacetylating agent or a pharmaceutical composition comprising a histone hyperacetylating agent for use in the treatment of varicosis. The use is focused/limited to the treatment of varicosis caused by the consequences of a genetic disorder, especially an epigenetic disorder.

*"Varicosis"* or *"Varicose Veins"* is a symptom in which the veins become enlarged and often highly visible directly under the skin. The disorder seems to develop over time mostly in the lower legs due to a genetic disorder.

*"Treatment of Varicosis"* is defined as a therapeutical effect on the varicosis, including ameliorating, strongly ameliorating or even curing the symptoms, including e.g. the disappearance of the varicose veins. Thus, it could also include curing the disease by removing the cause up to gene repair removing the epigenetic disorder. It might also apply to prophylactic approaches and thus *"Treatment of Varicosis"* would also encompass the prevention of varicosis caused by a genetic disorder or epigenetic disorder

In one embodiment the histone hyperacetylating agent itself or being comprised in the pharmaceutical composition used in the treatment of varicosis is a histone deacetylase inhibitor, preferably is selected from the group consisting of Trichostatin A and Trichostatin C; phenyl-butyrate; the group consisting of Oxamflati, Trapoxin A, FR901228, Apicidin, HC-Toxin, WF27082, and Chlamydocin; the group consisting of Salicylihydroxamic Acid, Suberoylanilide Hydroxamic Acid, and Azelaic Bishydroxamic Acid; the group consisting of Azelaic-I-Hydroxamate-9-Anilide, M-Carboxycinnamic Acid Bishydroxamide, 6-(3-Chlorophenylureido)carpoicHydroxamic Acid, MW2796, and MW2996; the group consisting of Sodium Butyrate, Isovalerate, Valerate, 4-Phenylbutyrate, Phenylbutyrate, Propionate, Butrymide, Isobutyramide, Phenylacetate, 3-Bromopropionate, and Tributyrin; the group consisting of MS-27-275 and the 3'-amino derivative thereof; or the group consisting of Depudecin and Scriptaid or is Vorinostat or other related Hydroxamic Acids; or from those new histone deacetylase inhibitors listed in the article "New patented histone deacetylase inhibitors" by Wang H. and Dymock BW (Expert Opin. Ther. Patents (2009) 19(12):1727-1757) like AR-42, belinostat (PXD101), benzamides, chidamide (CS055/HBI8000), CHR-2845, CUDC-101, cyclic peptides, depsipeptide, entinostat (MS-275), givinostat (ITF2357), hydroxamates, JNJ26481585, KD-5170, mocetinostat (MGCD0103), panobinostat (LBH589), PCI-24781 (CRA-024781), resminostat (4SC-201, BYK408740), romidepsin (FK228), SB939, or vorinostat (SAHA).

In a very preferred embodiment the histone hyperacetylating agent itself or being comprised in the pharmaceutical composition used in the treatment of varicosis is an active ingredient A selected from a phenyl-butyric acid, derivative or physiologically acceptable salt thereof, preferably being selected from 4-phenyl-butyric acid or a physiologically acceptable salt thereof, more preferably being a physiologically acceptable salt of 4-phenyl-butyric acid, more preferably being a physiologically acceptable salt of 4-phenyl-butyric acid of the earth or earth alkaline type, most preferably is a lithium, sodium, magnesium or calcium, preferably the sodium salt of 4-phenyl-butyric acid.

In another preferred embodiment of the histone hyperacetylating agent or the pharmaceutical composition comprising it used in the treatment of varicosis, the treatment is done by treating the patient by administration of between 80 to 4000 mg/day, or 200 to 2000 mg/day or a maximum of 1000 mg/day of active ingredient A.

In another preferred embodiment of the histone hyperacetylating agent or the pharmaceutical composition comprising it, used in the treatment of varicosis the treatment is done by treating the patient by oral administration of active ingredient A or the pharmaceutical composition, preferably by once or twice daily oral administration of active ingredient A or the pharmaceutical composition.

A highly important aspect of the invention rests in that the use of the active ingredient A (the histone hyperacetylating agent) or of the pharmaceutical composition comprising it, is done in a way to ensure a constant level (concentration) of the active ingredient A (phenyl-butyric acid, derivative or physiologically acceptable salt thereof) in the blood.

Accordingly, in another preferred embodiment the active ingredient A (the histone hyperacetylating agent) used in the treatment of varicosis is comprised in a pharmaceutical composition being a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC). Other options could include pharmaceutical composition being oral pharmaceutical unit dosage forms with an enteric coating or being based on nanoparticles to ensure a constant blood level of the active ingredient A. Very preferred embodiments of the oral pharmaceutical composition to be used according to the invention and comprising 4-phenyl-butyric acid or a physiologically acceptable salt thereof are disclosed in EP1427396 (WO2003/22253), the content of which forming herewith part of the description/disclosure of the current invention. In using this oral pharmaceutical composition, the active ingredient A is applied twice daily with 40 to 2000 mg/day, or 100 to 1000 mg/day or twice daily with 250 mg/day of the active ingredient A (like 4-phenyl-butyric acid or sodium phenylbutyrate).

Another of the very much preferred aspects/embodiments of this invention relates to a histone hyperacetylating agent or a pharmaceutical composition comprising a histone hyperacetylating agent for use in the treatment of vaginitis. The use is focused/limited to the treatment of vaginitis caused by the consequences of a genetic disorder, especially an epigenetic disorder.

*"Vaginitis"* is an inflammatory process of the vagina caused by a bacterial inflammation. It usually is accompanied by discharge as well as by itching and pain. It includes the chronic recurrent yeast vaginitis.

*"Treatment of Vaginitis"* is defined as a therapeutical effect on the vaginitis, including ameliorating, strongly ameliorating or even curing the symptoms, including e.g. the disappearance of discharge as well as of the itching and pain. Thus could also include curing the disease by removing the cause up to gene repair removing the epigenetic disorder. It might also apply to prophylactic approaches and thus *"Treatment of Vaginitis"* would also encompass the prevention of vaginitis caused by a genetic disorder or epigenetic disorder.

In one embodiment the histone hyperacetylating agent itself or being comprised in the pharmaceutical composition used in the treatment of vaginitis is a histone deacetylase inhibitor, preferably is selected from the group consisting of Trichostatin A and Trichostatin C; phenyl-butyrate; the group consisting of Oxamflati, Trapoxin A, FR901228, Apicidin, HC-Toxin, WF27082, and Chlamydocin; the group consisting of Salicylihydroxamic Acid, Suberoylanilide Hydroxamic Acid, and Azelaic Bishydroxamic Acid; the group consisting of Azelaic-I-Hydroxamate-9-Anilide, M-Carboxycinnamic Acid Bishydroxamide, 6-(3-Chlorophenylureido)carpoicHydroxamic Acid, MW2796, and MW2996; the group consisting of Sodium Butyrate, Isovalerate, Valerate, 4-Phenylbutyrate, Phenylbutyrate, Propionate, Butrymide, Isobutyramide, Phenylacetate, 3-Bromopropionate, and Tributyrin; the group consisting of MS-27-275 and the 3'-amino derivative thereof; or the group consisting of Depudecin and Scriptaid or is Vorinostat or other related Hydroxamic Acids; or from those new histone deacetylase inhibitors listed in the article "New patented histone deacetylase inhibitors" by Wang H. and Dymock BW (Expert Opin. Ther. Patents (2009) 19(12):1727-1757) like AR-42, belinostat (PXD101), benzamides, chidamide (CS055/HBI8000), CHR-2845, CUDC-101, cyclic peptides, depsipeptide, entinostat (MS-275), givinostat (ITF2357), hydroxamates, JNJ26481585, KD-5170, mocetinostat (MGCD0103), panobinostat (LBH589), PCI-24781 (CRA-024781), resminostat (4SC-201, BYK408740), romidepsin (FK228), SB939, or vorinostat (SAHA).

In a very preferred embodiment the histone hyperacetylating agent itself or being comprised in the pharmaceutical composition used in the treatment of vaginitis is an active ingredient A selected from a phenyl-butyric acid, derivative or physiologically acceptable salt thereof, preferably being selected from 4-phenyl-butyric acid or a physiologically acceptable salt thereof, more preferably being a physiologically acceptable salt of 4-phenyl-butyric acid, more preferably being a physiologically acceptable salt of 4-phenyl-butyric acid of the earth or earth alkaline type, most preferably is a lithium, sodium, magnesium or calcium, preferably the sodium salt of 4-phenyl-butyric acid.

In another preferred embodiment of the histone hyperacetylating agent or the pharmaceutical composition comprising it used in the treatment of vaginitis the treatment is done by treating the patient by administration of 80 to 4000 mg/day, or 200 to 1000 mg/day or approximately 500 mg/day of the active ingredient A.

In another preferred embodiment of the histone hyperacetylating agent or the pharmaceutical composition comprising it, used in the treatment of vaginitis the treatment is done by treating the patient by oral administration of active ingredient A or the pharmaceutical composition, preferably by once or twice daily oral administration of active ingredient A or the pharmaceutical composition.

A highly important aspect of the invention rests in that the use of the active ingredient A (the histone hyperacetylating agent) or of the pharmaceutical composition comprising it, is done in a way to ensure a constant level (concentration) of the active ingredient A (phenyl-butyric acid, derivative or physiologically acceptable salt thereof) in the blood.

Accordingly, in another preferred embodiment the active ingredient A (the histone hyperacetylating agent) used in the treatment of vaginitis is comprised in a pharmaceutical composition being a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC). Other options could include pharmaceutical composition being oral pharmaceutical unit dosage forms with an enteric coating or being based on nanoparticles to ensure a constant blood level of the active ingredient A. Very preferred embodiments of the oral pharmaceutical composition to be used according to the invention and comprising 4-phenyl-butyric acid or a physiologically acceptable salt thereof are disclosed in EP1427396 (WO2003/22253), the content of which forming herewith part of the description/disclosure of the current invention. In using this oral pharmaceutical composition, the active ingredient A is applied twice daily with 40 to 2000 mg/day, or 100 to 1000 mg/day or twice daily with 250 mg/day of the active ingredient A (like 4-phenyl-butyric acid or sodium phenylbutyrate).

Another of the very much preferred aspects/embodiments of this invention relates to a histone hyperacetylating agent or a pharmaceutical composition comprising a histone hyperacetylating agent for use in the prevention of Sudden Infant Death Syndrome. The use is focused/limited to the prevention of Sudden Infant Death Syndrome explicable through the consequences of a - likely undiscovered - genetic disorder, especially an epigenetic disorder.

*"Sudden Infant Death Syndrome"* is defined as a syndrome marked by the sudden death of an infant that is unexpected by history, in which a child is put to sleep and unexpectedly dies. Underlying the claim for the prevention of this syndrome is the understanding that the cause of a number of cases rated by the involved physician under *"Sudden Infant Death Syndrome"* which by standard definition would remain inexplicable even after thorough post-mortem analysis are explicable through the consequences of an undiscovered genetic disorder, especially an epigenetic disorder.

*"Prevention of Sudden Infant Death Syndrome"* is defined as lowering the risk of occurrence of "Sudden Infant Death Syndrome" by ameliorating, strongly ameliorating or even curing the symptoms caused by the underlying genetic disorder and also includes removing the cause up to gene repair like removing the epigenetic disorder.

In one embodiment the histone hyperacetylating agent itself or being comprised in the pharmaceutical composition used in the prevention of Sudden Infant Death Syndrome is a histone deacetylase inhibitor, preferably is selected from the group consisting of Trichostatin A and Trichostatin C; phenyl-butyrate; the group consisting of Oxamflati, Trapoxin A, FR901228, Apicidin, HC-Toxin, WF27082, and Chlamydocin; the group consisting of Salicylihydroxamic Acid, Suberoylanilide Hydroxamic Acid, and Azelaic Bishydroxamic Acid; the group consisting of Azelaic-I-Hydroxamate-9-Anilide, M-Carboxycinnamic Acid Bishydroxamide, 6-(3-Chlorophenylureido)carpoicHydroxamic Acid, MW2796, and MW2996; the group consisting of Sodium Butyrate, Isovalerate, Valerate, 4-Phenylbutyrate, Phenylbutyrate, Propionate, Butrymide, lsobutyramide, Phenylacetate, 3-Bromopropionate, and Tributyrin; the group consisting of MS-27-275 and the 3'-amino derivative thereof; or the group consisting of Depudecin and Scriptaid or is Vorinostat or other related Hydroxamic Acids; or from those new histone deacetylase inhibitors listed in the article "New patented histone deacetylase inhibitors" by Wang H. and Dymock BW (Expert Opin. Ther. Patents (2009) 19(12):1727-1757) like AR-42, belinostat (PXD101), benzamides, chidamide (CS055/HBI8000), CHR-2845, CUDC-101, cyclic peptides, depsipeptide, entinostat (MS-275), givinostat (ITF2357), hydroxamates, JNJ26481585, KD-5170, mocetinostat (MGCD0103), panobinostat (LBH589), PCI-24781 (CRA-024781), resminostat (4SC-201, BYK408740), romidepsin (FK228), SB939, or vorinostat (SAHA).

In a very preferred embodiment the histone hyperacetylating agent itself or being comprised in the pharmaceutical composition used in the prevention of Sudden Infant Death Syndrome is an active ingredient A selected from a phenyl-butyric acid, derivative or physiologically acceptable salt thereof, preferably being selected from 4-phenyl-butyric acid or a physiologically acceptable salt thereof, more preferably being a physiologically acceptable salt of 4-phenyl-butyric acid, more preferably being a physiologically acceptable salt of 4-phenyl-butyric acid of the earth or earth alkaline type, most preferably is a lithium, sodium, magnesium or calcium, preferably the sodium salt of 4-phenyl-butyric acid.

In another preferred embodiment of the histone hyperacetylating agent or the pharmaceutical composition comprising it used in the prevention of Sudden Infant Death Syndrome, the prevention is achieved by treating a child (weighing less than 20 kg) by administration of an amount of active ingredient A less than 450 mg/kg/day.

In another preferred embodiment of the histone hyperacetylating agent or the pharmaceutical composition comprising it used in the prevention of Sudden Infant Death Syndrome the prevention is achieved by treating a child by oral administration of active ingredient A or the pharmaceutical composition, preferably by once or twice daily oral administration of active ingredient A or the pharmaceutical composition.

A highly important aspect of the invention rests in that the use of the active ingredient A (the histone hyperacetylating agent) or of the pharmaceutical composition comprising it is done in a way to ensure a constant level (concentration) of the active ingredient A (phenyl-butyric acid, derivative or physiologically acceptable salt thereof) in the blood.

Accordingly, in another preferred embodiment the active ingredient A (the histone hyperacetylating agent) used in the prevention of Sudden Infant Death Syndrome is comprised in a pharmaceutical composition being a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC). Other options could include pharmaceutical composition being oral pharmaceutical unit dosage forms with an enteric coating or being based on nanoparticles to ensure a constant blood level of the active ingredient A. Very preferred embodiments of the oral pharmaceutical composition to be used according to the invention and comprising 4-phenyl-butyric acid or a physiologically acceptable salt thereof are disclosed in EP1427396 (WO2003/22253), the content of which forming herewith part of the description/disclosure of the current invention. In using this oral pharmaceutical composition, the active ingredient A is applied twice daily with 40 to 2000 mg/day, or 100 to 1000 mg/day or twice daily with 250 mg/day of the active ingredient A (like 4-phenyl-butyric acid or sodium phenylbutyrate).

Without limitation the invention is further described by way of examples below:

### Examples:

### Example 1: Patient No. 1 with Varicose Veins

A patient suffering from varicose veins was enrolled in a trial. After having been treated orally twice daily with 250mg of 4-phenylbutyrate sodium salt for 2 years, it was found out that the varicose veins did completely disappear.

### Example 2: Patient No. 2 with Vaginitis

A patient showing symptoms of vaginitis was treated in a trial. She severely suffered from constant discharge. After having been treated orally twice daily with 250mg of 4-phenylbutyrate sodium salt the discharge did significantly diminish until it completely disappeared after about 6 months of treatment.

### Example 3: Slow Release Tablets with 250 mg of Sodium 4-Phenylbutyrate

A mixture of 6,000.0 g of sodium 4-phenylbutyrate, 6,280.0 g of lactosum monohydricum, 3,500.0 g of Methocel K100 MPremium, and 750.0 g of Avicel PH 102 is wettened with 4,000.0 g of aqua purificata (water purified by inversion osmosis) and dried in cold air during 18 hours. The mixture is forced through a sieve IV mm and dried again during 10 hours with air of 40°C. A mixture of 240.0 g of talcum and 30.0 g of magnesium stearate is admixed during 20 minutes and the mixture is pressed into tablets of 0.70 g each, a thickness of about 6.8 mm and with a hardness of 90 Newton. Yield: 24,000 tablet cores.

The mixing is carried out with a Diosna Mixer, the drying in a Lükon drying cabinet, the sieving with a Köhler & Bosshard sieving machine, and the tablet pressing with a Korsch tablet press EK 11. The cores are provided with a film coating for example by using a colloidal dispersion containing 7,850 g of isopropylalcohol, 3,360 g of Eudragit L 12.5, 66 g of dibutyl phthalat, 18.0 g of Miglyol 812, and 56 g of polyethylenglycol PEG 400. The suspension is sprayed at 3.5 atü and 25° C onto the 24,000 cores. The film-coated tablets are dried in a circulating air drying cabinet for at least 4 hours at 35°C.

## Claims

1. A pharmaceutical composition comprising an active ingredient A selected from a phenyl-butyric acid a derivative or a physiologically acceptable salt thereof for use in the treatment of a genetic disorder.

2. The pharmaceutical composition used according to claim 1, wherein the genetic disorder is an epigenetic disorder.

3. The pharmaceutical composition used according to any of claims 1 or 2, wherein the genetic disorder is selected from or manifests itself in symptoms or diseases selected from urea cycle disorders, thalassemia, cystic fibrosis, rheumatoid arthritis, Siogren' s syndrome, uveitis, polymyositis, and dermatomyositis, arteriosclerosis, amyotrophic lateral sclerosis, asociality, affective disorders, systemic lupus erythematosus, immune response, varicosis, vaginitis, including chronic recurrent yeast vaginitis, depression or Sudden Infant Death Syndrome, preferably selected from depression, varicosis, vaginitis or Sudden Infant Death Syndrome.

4. The pharmaceutical composition according to any of claims 1 to 3, comprising an active ingredient A selected from a phenyl-butyric acid or a physiologically acceptable salt thereof for use in the treatment of a genetic disorder by administration of a maximum of 1000 mg/day of the active ingredient A, whereas the genetic disorder is an epigenetic disorder.

5. The pharmaceutical composition used according to claim 4, wherein the treatment is applied by oral administration of the pharmaceutical composition, preferably is applied by once or twice daily oral administration of the pharmaceutical composition.

6. The pharmaceutical composition used according to any of claims 4 or 5, wherein the treatment is applied to a child, preferably is applied to a child (weighing less than 20 kg) by administration of an amount of active ingredient A less than 450 mg/kg/day.

7. The pharmaceutical composition used according to any of claims 4 or 5, wherein the treatment is applied by administration of a maximum of between 750 and 250 mg/day of active ingredient A, preferably of between 600 and 400 mg/day of active ingredient A, most preferably of approximately or a maximum of 500 mg/day of active ingredient A.

8. The pharmaceutical composition used according to any of claims 4 to 7, wherein the active ingredient A is 4-phenyl-butyric acid or a physiologically acceptable salt thereof, preferably is a physiologically acceptable salt of 4-phenyl-butyric acid, more preferably is a physiologically acceptable salt of 4-phenyl-butyric acid of the earth or earth alkaline type, most preferably is a lithium, sodium, magnesium or calcium, preferably the sodium salt of 4-phenyl-butyric acid.

9. The pharmaceutical composition used according to any of claims 5 to 8, wherein the pharmaceutical composition is a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC).

10. A histone hyperacetylating agent or a pharmaceutical composition comprising it for use in the treatment of depression

11. A histone hyperacetylating agent or a pharmaceutical composition comprising it for use in the treatment of vaginitis.

12. A histone hyperacetylating agent or a pharmaceutical composition comprising it for use in the treatment of varicosis.

13. A histone hyperacetylating agent or a pharmaceutical composition comprising it for use in the prevention of Sudden Infant Death Syndrome.

14. The histone hyperacetylating agent used according to any of claims 10 to 13 or comprised in a pharmaceutical composition used according to any of claims 10 to 13 being an active ingredient A selected from a phenyl-butyric acid, derivative or physiologically acceptable salt thereof, preferably being selected from 4-phenyl-butyric acid or a physiologically acceptable salt thereof, more preferably being a physiologically acceptable salt of 4-phenyl-butyric acid, more preferably being a physiologically acceptable salt of 4-phenyl-butyric acid of the earth or earth alkaline type, most preferably is a lithium, sodium, magnesium or calcium, preferably the sodium salt of 4-phenyl-butyric acid.

15. The pharmaceutical composition used according to any of claims 10 to 14 being a prolonged release oral pharmaceutical unit dosage form, preferably comprising a controlled release agent, preferably selected from the group consisting of cellulose derivatives, polyvinyl chloride, polyvinyl acetate, polyethylene, or polymers or co-polymers of acrylic acid esters and methyl-acrylic acid esters, more preferably selected from cellulose derivatives like ethylcellulose, methylcellulose, ethylhydroxyethylcellulose, ethylcellulose, hyhydroxypropylmethylcellulose (HPMC) or hydroxypropylcellulose (HPC).
